# FASCICULE DE BREVET EUROPEEN

(11) **EP 0 626 166 B1**
(45) Date de publication et mention de la délivrance du brevet: **19.03.1997**
(21) Numéro de dépôt: 94401148.5
(22) Date de dépôt: 25.05.1994
(51) Int. Cl.: A61K 7/09

(54) **Nouveau procédé de déformation permanente des cheveux et composition pour sa mise en oeuvre contenant en association un amino ou amidothiol et au moins un bromure minéral**
Neues Verfahren zur dauerhaften Haarverformung und Mittel zur Durchführung dieses Verfahrens, die eine Kombination aus einem Amino oder Amidothiol und mindestens einem mineralischen Bromid enthalten
New process for permanent waving of hair and the composition therefor containing in association an amino or amidothiol and at least a mineral bromide

(30) Priorité: 25.05.1993 FR 9306218
(43) Date de publication de la demande: 30.11.1994
(73) Titulaire: L'OREAL, 75008 Paris (FR)
(72) Inventeur: Leroy, Frédéric, F-92210 Saint Cloud (FR); Malle, Gérard, F-77580 Villiers sur Morin (FR); Burande, Agnès, F-77270 Vileparisis (FR); Duvault, Yolanda, F-93600 Aulnay-sous-Bois (FR)
(74) Mandataire: Stalla-Bourdillon, Bernard

(56) Documents cités:
- EP-A- 0 076 444
- EP-A- 0 443 356
- WO-A-91/02538
- DE-A- 1 959 149
- DE-A- 3 022 049

## Description

La présente invention a pour objet un nouveau procédé de déformation permanente des cheveux consistant, prélablement ou simultanément, à l'étape de réduction à l'aide d'un agent réducteur du type amino ou amidothiol, à appliquer sur les cheveux un bromure minéral en tant qu'agent dopant. La présente invention a également pour objet une composition cosmétique contenant en association un amino ou amidothiol et un bromure minéral pour la réalisation de la première étape d'une opération de déformation permanente des cheveux.

La technique pour réaliser la déformation permanente des cheveux consiste, dans un premier temps, à réaliser l'ouverture des liaisons disulfures de la kératine (cystine) à l'aide d'une composition contenant un agent réducteur (étape de réduction), puis après avoir de préférence rincé la chevelure, à reconstituer dans un second temps lesdites liaisons disulfures en appliquant, sur les cheveux sous tension, une composition oxydante, (étape d'oxydation, dite aussi de fixation) de façon à donner aux cheveux la forme recherchée. Cette technique permet indifféremment de réaliser soit l'ondulation des cheveux, soit leur défrisage ou leur décrêpage.

Les compositions pour réaliser le premier temps d'une opération de permanente se présentent généralement sous forme de lotions, de crèmes, de gels ou de poudres à diluer dans un support liquide et contiennent, en tant qu'agent réducteur, de préférence un thiol.

Parmi ces derniers, les produits les plus couramment utilisés sont l'acide thioglycolique, le monothioglycolate de glycérol et la cystéine.

Ces agents réducteurs et notamment l'acide thioglycolique, qui est généralement considéré comme le produit de référence pour ces compositions pour permanente, réduisent efficacement les liaisons disulfures de la kératine.

Toutefois, l'acide thioglycolique ne permet d'obtenir une frisure de qualité suffisante que lorsqu'il est utilisé en milieu suffisamment basique c'est-à-dire à pH supérieur à 8,5.

Le monothioglycolate de glycérol, dont le pH optimum est plus proche de la neutralité, produit une qualité de frisure inférieure à celle obtenue avec l'acide thioglycolique.

La cystéine, généralement utilisée sous forme de L-cystéine base ou de son chlorhydrate, présente l'avantage d'être considérablement moins malodorante que les deux composés précédents mais son faible pouvoir réducteur ne permet pas d'obtenir une frisure d'intensité et de tenue satisfaisantes. De plus, la cystéine doit être mise en oeuvre à un pH très alcalin (supérieur à 9,0), ce qui influe sur la dégradation du cheveu et augmente le pouvoir irritant de la composition.

Il est par conséquent souhaitable d'améliorer les performances des agents réducteurs conventionnels tout en supprimant leurs inconvénients. Le terme d'amélioration désigne à la fois l'amélioration des qualités de frisure (importance, tenue, beauté) obtenue en utilisant la même quantité de produit, l'obtention d'une qualité de frisure comparable en utilisant une quantité moindre de produit, mais également l'obtention de la même qualité de frisure dans des conditions plus satisfaisantes telles que par exemple à un pH plus proche de la neutralité rendant le traitement moins dégradant pour le cheveu.

Divers moyens pour améliorer la qualité et la tenue de la frisure ont été proposés. Parmi ceux-ci, on peut citer notamment l'augmentation du temps de pose, l'apport de chaleur, la mise en oeuvre à un pH plus élevé. Ces moyens sont efficaces mais s'accompagnent malheureusement d'une dégradation plus importante du cheveu.

Il a également été proposé l'utilisation d'agents de gonflement des cheveux tels que les formamides et en particulier d'urée, d'alcanediols, ou encore des éthers de glycols, ce qui favorise la pénétration des agents réducteurs dans le cheveu et permet d'obtenir une certaine amélioration des performances des agents réducteurs, mais celle-ci est très insuffisante à l'égard des amino et amidothiols et leurs dérivés tels que la cystéine.

On décrit une composition pour la déformation permanente des cheveux qui contient un thioglycolate de glycérol et un bromure.

On a maintenant constaté, de façon tout à fait surprenante, que si les agents réducteurs de la kératine du type amino ou amidothiol étaient associés à un bromure minéral dans des proportions bien définies, il était possible d'obtenir un effet de dopage de l'agent réducteur se traduisant d'une part par une augmentation importante de l'intensité et de la tenue de la frisure et d'autre part par la possibilité de mettre en oeuvre à un pH neutre des amino ou amidothiols fonctionnant normalement à pH alcalin. Cette amélioration des propriétés n'est accompagnée d'aucune dégradation supplémentaire de l'état de la fibre des cheveux ce qui est par conséquent très satisfaisant sur le plan cosmétique.

La présente invention a donc pour objet, un procédé de déformation permanente des cheveux consistant, dans une première étape de réduction, à réaliser l'ouverture des liaisons kératiniques des cheveux par application d'un agent réducteur choisi parmi au moins un amino ou amidothiol ou un de leurs sels puis dans une deuxième étape d'oxydation, à reformer lesdites liaisons à l'aide d'une composition oxydante ou par action de l'oxygène de l'air, ledit procédé étant essentiellement caractérisé par le fait que, préalablement ou simultanément à l'application de l'agent réducteur, on applique sur les cheveux, en tant qu'agent dopant dudit agent réducteur, au moins un bromure alcalin, alcalino-terreux ou d'ammonium.

Les amino ou amidothiols utilisables selon l'invention peuvent être représentés par les formules (I) et (II) qui seront définies ci-après.

Parmi les bromures alcalins et alcalino-terreux utilisables selon l'invention, on peut citer notamment les bromures de sodium, de potassium et de calcium.

Selon un premier mode de réalisation du procédé selon l'invention, l'agent dopant est appliqué préalablement à l'étape de réduction pendant 1 à 15 minutes, sous la forme d'une composition de prétraitement dans laquelle il est présent en une concentration comprise entre 1 et 30 % en poids par rapport au poids total de la composition.

La composition de prétraitement est de préférence une solution aqueuse dont le pH est de préférence compris entre 4 et 11.

Selon un second mode de réalisation de l'invention, l'agent dopant est appliqué sur les cheveux en association avec l'agent réducteur sous forme d'une composition dans laquelle le bromure est présent en une concentration comprise entre 1 et 30 % en poids par rapport au poids total de la composition.

Selon différentes variantes de l'invention, le procédé de déformation permanente des cheveux peut être un procédé d'ondulation, de défrisage ou de décrêpage des cheveux.

Lorsque le procédé de déformation permanente des cheveux est un procédé d'ondulation, les cheveux, à l'état mouillé, sont enroulés autour de rouleaux de 4 à 20 mm de diamètre et l'on applique la composition réductrice, précédée éventuellement de l'application de la composition de prétraitement. La composition réductrice peut éventuellement être appliquée au fur et à mesure de l'enroulage des cheveux.

On laisse ensuite agir la composition réductrice pendant 5 à 60 minutes et de préférence 5 à 30 minutes à une température comprise entre 20 et 55°C puis rince abondamment, après quoi on applique sur les cheveux enroulés une composition oxydante, permettant de reformer les liaisons disulfures de la kératine, pendant un temps de pose de 2 à 10 minutes. On peut également laisser agir l'oxygène de l'air.

Puis on enlève les rouleaux et on rince abondamment la chevelure.

Lorsque le procédé de déformation permanente des cheveux est un procédé de défrisage ou de décrêpage, on applique sur les cheveux la composition réductrice, éventuellement précédée de la composition de prétraitement, puis on soumet les cheveux à une déformation mécanique permettant de les fixer dans leur nouvelle forme, par une opération de lissage des cheveux avec un peigne à large dents, avec le dos d'un peigne ou à la main.

Après un temps de pose de 5 à 60 minutes, en particulier de 5 à 30 minutes, on procède alors à un nouveau lissage puis on rince soigneusement et on applique la composition oxydante ou fixatrice que l'on laisse agir pendant un temps de 2 à 10 minutes environ puis on rince abondamment les cheveux.

La présente invention a également pour objet, à titre de produit industriel nouveau, une composition cosmétique pour le premier temps d'une opération de déformation permanente des cheveux pour la mise en oeuvre du procédé selon l'invention contenant, dans un véhicule cosmétique approprié, en tant que seul et unique agent réducteur au moins un amino ou amidothiol ou un de leurs sels et en tant qu'agent dopant dudit agent réducteur au moins un bromure alcalin, alcalino-terreux ou d'ammonium.

Le bromure alcalin ou alcalino-terreux de la composition cosmétique selon l'invention, peut être comme mentionné ci-dessus le bromure de sodium, le bromure de potassium et le bromure de calcium.

Le bromure est généralement présent en une concentration comprise entre 1 et 30 % en poids par rapport au poids total de la composition cosmétique.

Dans la composition cosmétique selon l'invention, le rapport molaire entre l'agent dopant et l'agent réducteur est de préférence compris entre 0,1 et 2,5.

Selon l'invention, les amino et amidothiols utilisés en tant qu'agents réducteurs de la composition réductrice peuvent être représentés par les formules (I) et (II) suivantes :

HS-A-NR₁R₂ (I)

dans laquelle :
- A représente :: (a) le radical divalent -(CH₂)ₙ-, n étant un nombre entier compris entre 2 et 5,
(b) le radical divalent -(CH₂)₂-O-(CH₂)₂-, ou
(c) le radical divalent 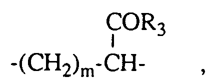 m étant 1 ou 2, R₃ étant (i) le radical OH ou (ii) le radical OR₄, R₄ représentant un radical alkyle inférieur, linéaire ou ramifié, ayant de 1 à 3 atomes de carbone ou (iii) le radical -NR₅R₆, R₅ et R₆, identiques ou différents, représentant soit un atome d'hydrogène soit un radical alkyle inférieur, linéaire ou ramifié, ayant de 1 à 3 atomes de carbone,
R₁ et R₂ représentent, indépendamment l'un de l'autre, un atome d'hydrogène, un radical alkyle inférieur, linéaire ou ramifié, ayant de 1 à 4 atomes de carbone, ou un radical -COR₇, R₇ étant un radical alkyle inférieur, linéaire ou ramifié, ayant de 1 à 4 atomes de carbone, et

HS-(CH₂)ₚ-NR₈R₉ (II)

dans laquelle :
p est 2 ou 3
- R₈ représente: (a) le radical -CO-(CH₂)ₜ-OH, t étant un nombre entier compris entre 2 et 5,
(b) le radical -CO-(CHOH)₄-CH₂OH, ou
(c) le radical 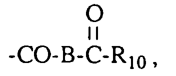 R₁₀ représentant un radical hydroxy,
- B représente: (i) le radical divalent -(CH₂)_{q}-, q étant 2 ou 3,
(ii) le radical divalent 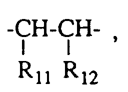 R₁₁ et R₁₂, identiques ou différents,
représentant un radical alkyle, linéaire ou ramifié, ayant de 1 à 4 atomes de carbone ou bien R₁₁ et R₁₂ forment ensemble, avec les atomes de carbone adjacents, un cycle cyclohexane, ou
(iii) le radical divalent 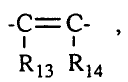 R₁₃ et R₁₄, identiques ou différents,
représentent un atome d'hydrogène, un radical alkyle inférieur, linéaire ou ramifié, ayant de 1 à 4 atomes de carbone, ou bien R₁₃ et R₁₄ forment ensemble, avec les atomes de carbone adjacents, un cycle benzénique, et
R₉ représente un atome d'hydrogène, ou R₉ et R₁₀ pris ensemble forment une liaison simple.

Par radical alkyle inférieur, linéaire ou ramifié, ayant de 1 à 4 atomes de carbone, on doit entendre un radical méthyle, éthyle, isopropyle, butyle, isobutyle ou méthyl-1 propyle.

Parmi les agents réducteurs de formules (I) et (II) ci-dessus, on peut notamment citer les suivants :
- la cystéine,
- le cystéinate de méthyle,
- le cystéinate d'éthyle,
- la N-acétyl cystéine,
- la mercapto-2 éthylamine,
- la mercapto-3 propylamine,
- la mercapto-5 pentylamine,
- la N-acétyl cystéamine,
- la N-propionyl cystéamine,
- la N-butyryl cystéamine,
- la N-isobutyryl cystéamine,
- l'acide N-(mercapto-2 éthyl) succinamique et
- le N-(mercapto-2 éthyl) succinimide.

L'agent réducteur selon l'invention tel que défini ci-dessus peut éventuellement être utilisé sous forme de sel.

Parmi les sels cosmétiquement acceptables des composés des formules (I) et (II) ceux particulièrement préférés sont les chlorhydrates, les bromhydrates, les citrates, les oxalates, les lactates et les acétates.

Les composés des formules (I) et (II) sont pour la plupart connus et pour certains, ont été décrits dans les demandes FR 90.08343, FR 89.15182, FR 91.06029 et dans la demande de brevet européen 89.402209.

Dans les compositions réductrices selon l'invention, l'agent réducteur tel que défini ci-dessus est généralement présent à une concentration comprise entre 1 et 30 % et de préférence entre 5 et 20 % en poids par rapport au poids total de la composition réductrice.

Le pH des compositions réductrices selon l'invention est de préférence compris entre 4 et 11 et plus particulièrement entre 6 et 10, ledit pH étant obtenu à l'aide d'un agent alcalin tel que l'ammoniaque, la monoéthanolamine, la diéthanolamine, la triéthanolamine, la propanediamine-1,3, un carbonate ou un bicarbonate alcalin ou d'ammonium, un carbonate organique tel que le carbonate de guanidine, un hydroxyde alcalin, ou à l'aide d'un agent acidifiant tel que par exemple l'acide chlorhydrique, l'acide bromhydrique, l'acide acétique, l'acide lactique, l'acide oxalique ou l'acide borique ou à l'aide de tampons tels que par exemple les phosphates mono et dipotassique.

Selon un mode de réalisation préféré, les compositions réductrices contiennent également un agent tensioactif de type non-ionique, anionique, cationique ou amphotère et parmi ceux-ci, on peut citer les alkylsulfates, les alkylbenzènesulfonates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Lorsque les compositions réductrices contiennent au moins un agent tensioactif, celui-ci est généralement présent à une concentration maximale de 30 % en poids, mais de préférence comprise entre 0,5 et 10 % en poids par rapport au poids total de la composition réductrice.

Dans le but d'améliorer les propriétés cosmétiques des cheveux ou encore d'en atténuer ou d'éviter leur dégradation, les compositions réductrices peuvent également contenir un agent traitant de nature cationique, anionique, non-ionique ou amphotère.

Parmi les agents traitants particulièrement préférés, on peut notamment citer ceux décrits dans les brevets français n° 2.598.613, et n° 2.470.596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2.535.730, les polyorganosiloxanes à groupements aminoalkyles modifiés par des groupements alcoxycarbonylalkyles tels que ceux décrits dans le brevet US n°4.749.732, des polyorganosiloxanes tels que le copolymère polydiméthylsiloxanepolyoxyalkyle du type Diméthicone Copolyol, un polydiméthylsiloxane à groupements terminaux stéaroxy (stéaroxydiméthicone), un copolymère polydiméthylsiloxanedialkylammonium acétate ou un copolymère polydiméthyl-siloxane polyalkylbétaïne décrits dans le brevet britannique n° 2.197.352, des polysiloxanes organo modifiés par des groupements mercapto ou mercaptoalkyles tels que ceux décrits dans le brevet français n° 1.530.369 et dans la demande de brevet européen n° 295.780, ainsi que des silanes tels que le stéaroxytriméthylsilane.

Les compositions réductrices peuvent également contenir d'autres ingrédients traitants comme des polymères cationiques tels que ceux utilisés dans les compositions des brevets français n° 79.32078 (2.472.382) et 80.26421 (2.495.931), ou encore des polymères cationiques du type ionène tels que ceux utilisés dans les compositions du brevet luxembourgeois n° 83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO₂/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple, le monométhyléther de propylèneglycol, le monométhyléther de dipropylèneglycol, le monoéthyléther de l'éthylèneglycol et le monoéthyléther du diéthylèneglyol, des alcanediols en C₃-C₆ tels que par exemple le propanediol-1,2 et le butanediol-1,2, l'imidazolidinone-2 ainsi que d'autres composés tels que des alcools gras, des dérivés de la lanoline, des ingrédients actifs tels que l'acide pantothénique, des agents antichutes, des agents antipelliculaires, des épaississants, des agents de suspension, des agents séquestrants, des agents opacifiants, des colorants, des filtres solaires ainsi que des parfums et conservateurs.

Les compositions réductrices selon l'invention se présentent essentiellement sous forme aqueuse notamment sous la forme d'une lotion épaissie ou non, d'une crème ou d'un gel.

Les compositions réductrices selon l'invention peuvent être également du type exothermique, c'est-à-dire provoquant un certain échauffement lors de l'application sur les cheveux, ce qui apporte un agrément à la personne qui subit le premier temps de la permanente ou du défrisage.

Les compositions réductrices selon l'invention peuvent également contenir un solvant tel que par exemple de l'éthanol, du propanol, ou de l'isopropanol ou encore du glycérol à une concentration maximale de 20 % par rapport aux poids total de la composition.

Le véhicule des compositions réductrices selon l'invention est de préférence de l'eau ou une solution hydroalcoolique d'un alcool inférieur tel que l'éthanol, l'isopropanol ou le butanol.

Lorsque les compositions sont destinées à une opération de défrisage ou de décrêpage des cheveux, les compositions réductrices sont de préférence sous forme d'une crème de façon à maintenir les cheveux aussi raides que possible. On réalise ces crèmes sous forme d'émulsions "lourdes", par exemple à base de stéarate de glycéryle, de stéarate de glycol, de cires auto-émulsionnables, d'alcools gras, etc...

On peut également utiliser des liquides ou des gels contenant des agents épaississants tels que des polymères ou des copolymères carboxyvinyliques qui "collent" les cheveux et les maintiennent dans la position lisse pendant le temps de pose.

La composition d'oxydation ou oxydante mise en oeuvre dans le procédé de déformation permanente des cheveux selon l'invention est du type couramment utilisé et contient comme agent oxydant de l'eau oxygénée, un bromate alcalin, un persel, un polythionate ou un mélange de bromate alcalin et de persel.

La concentration en eau oxygénée peut varier de 1 à 20 volumes et de préférence de 1 à 10, la concentration en bromate alcalin de 2 à 12 % et celle en persel de 0,1 à 15 % en poids par rapport au poids total de la composition oxydante. Le pH de la composition oxydante est généralement compris entre 2 et 8 mais de préférence entre 3 et 6. L'eau oxygénée peut être stabilisée par exemple par la phénacétine, l'acétanilide, les phosphates mono ou trisodiques ou par le sulfate d'hydroxy-8 quinoléine. L'oxydation peut être immédiate ou différée.

On va maintenant donner à titre d'illustration plusieurs exemples du procédé de déformation permanente des cheveux, ainsi que de compositions réductrices selon l'invention.

### EXEMPLE 1

On prépare, selon l'invention, une composition réductrice de déformation permanente des cheveux en procédant au mélange des ingrédients suivants :

| | |
|---|---|
| - Cystéine | 12,1 g |
| - Bromure d'ammonium | 9,8 g |
| - Sel pentasodique de l'acide diéthylènetriamine penta-acétique | 0,15 g |
| - Parfum q.s. | |
| - Ammoniaque q.s. pH 8,5 | |
| - Eau déminéralisée q.s.p. | 100 g |

Cette composition est appliquée sur des cheveux mouillés préalablement enroulés sur des rouleaux de mise en plis. Après avoir laissé agir la composition pendant environ 15 min., on rince abondamment à l'eau puis on applique la composition oxydante suivante :

| | |
|---|---|
| - Eau oxygénée | 2 g |
| - Stannate de sodium | 0,015 g |
| - Lauryl sulfate d'ammonium | 1,4 g |
| - Acide citrique | 0,5 g |
| - Parfum q.s. | |
| - Eau déminéralisée q.s.p. | 100 g |

On laisse agir la composition oxydante pendant environ 5 min., puis on enlève les rouleaux, et rince abondamment la chevelure à l'eau. Après séchage sous casque, les cheveux présentent de belles boucles.

Dans cet exemple, la composition oxydante peut être remplacée par la composition suivante :

| | |
|---|---|
| - Bromate de sodium | 8 g |
| - Triéthanolamine q.s. pH 8,0 | |
| - Phosphate monosodique hydraté (12H₂0) | 0,3 g |
| - Phosphate trisodique hydraté | 0,5 g |
| - Cocoamidopropylbétaïne vendu sous la dénomination "Tegobétaïne HS" par la Société Goldschmidt | 1 g |
| - Parfum q.s. | |
| - Eau déminéralisée q.s.p. | 100 g |

### EXEMPLES 2 à 8

Selon le même mode de réalisation que décrit ci-dessus, on a procédé à des déformations permanentes des cheveux en remplaçant la composition réductrice de l'Exemple 1 par l'une des compositions réductrices *A* à *H* suivantes :

| *Composition A* : | |
|---|---|
| - Bromhydrate de cystéine | 22 g |
| - Bromure d'ammonium | 9,8 g |
| - Sel pentasodique de l'acide diéthylènetriamine penta-acétique | 0,15 g |
| - Parfum q.s. | |
| - Ammoniaque q.s. pH 8,5 | |
| - Eau déminéralisée q.s.p. | 100 g |

| *Composition B* : | |
|---|---|
| - Chlorhydrate de cystéamine | 11,5 g |
| - Bromure d'ammonium | 9,8 g |
| - Oxyde de laurylamine vendu sous la dénomination "Aromox DMMCD/W" par la Société Akzo | 2 g |
| - Acide éthylène diamine tétracétique | 0,15 g |
| - Parfum q.s. | |
| - Monoéthanolamine q.s. pH 8,0 | |
| - Eau déminéralisée q.s.p. | 100 g |

| *Composition C* : | |
|---|---|
| - Ester méthylique de cystéine | 13,5 g |
| - Bromure d'ammonium | 9,8 g |
| - Oxyde de laurylamine vendu sous la dénomination "Aromox DMMCD/W" par la Société Akzo | 1,2 g |
| - Parfum q.s. | |
| - Ammoniaque (20%) q.s. pH 7,2 | |
| - Eau déminéralisée q.s.p. | 100 g |

L'ester méthylique de cystéine doit être ajouté au moment de l'emploi.

| *Composition D* : | |
|---|---|
| - N-propionyl cystéamine | 13,2 g |
| - Bromure d'ammonium | 9,8 g |
| - Ester stéarique polyoxyéthyléné avec 8 moles d'oxyde d'éthylène vendu sous la dénomination "Myrj 45" par la Société ICI | 0,85 g |
| - Conservateur | 0,35 g |
| - Parfum q.s. | |
| - Ammoniaque q.s. pH 6,8 | |
| - Eau déminéralisée q.s.p. | 100 g |

| *Composition E* : | |
|---|---|
| - Acide N-(mercapto-2 éthyle) succinamique | 8,8 g |
| - Bromure d'ammonium | 10 g |
| - Chlorhydrate de cystéamine | 6,0 g |
| - Oxyde de laurylamine vendu sous la dénomination "Aromox DMMCD/W" par la Société Akzo | 2,0 g |
| - Conservateur | 0,15 g |
| - Parfum q.s. | |
| - Monoéthanolamine q.s. pH 8,5 | |
| - Eau déminéralisée q.s.p. | 100 g |

| *Composition F* : | |
|---|---|
| - Bromhydrate de cystéine | 22 g |
| - Bromure de sodium | 10 g |
| - Chlorure d'oléocétyl diméthyl hydroxyéthyl ammonium | 0,3 g |
| - Conservateur | 0,15 g |
| - Parfum q.s. | |
| - Ammoniaque q.s. pH 9,0 | |
| - Eau déminéralisée q.s.p. | 100 g |

| *Composition G* : | |
|---|---|
| - Cystéine | 12,1 g |
| - Bromure de potassium | 12 g |
| - Sel pentasodique de l'acide diéthylènetriamine penta-acétique | 0,15 g |
| - Parfum q.s. | |
| - Ammoniaque q.s. pH 8,5 | |
| - Eau déminéralisée q.s.p. | 100 g |

| *Composition H* : | |
|---|---|
| - N-propionyl cystéamine | 13 ,5 g |
| - Bromure de calcium hydraté | 11,1 g |
| - Ester stéarique polyoxyéthyléné avec 8 moles d'oxyde d'éthylène vendu sous la dénomination de "Myrj 45" par la Société ICI | 0,9 g |
| - Conservateur | 0,3 g |
| - Parfum q.s. | |
| - Ammoniaque q.s. pH 7,0 | |
| - Eau déminéralisée q.s.p. | 100 g |

### EXEMPLE 9

On prépare une composition de prétraitement en procédant au mélange des ingrédients suivants :

| | |
|---|---|
| - Bromure d'ammonium | 9,8 g |
| - Parfum q.s. | |
| - Ammoniaque q.s. pH 9 | |
| - Eau déminéralisée q.s.p. | 100 g |

Cette composition est appliquée sur des cheveux mouillés préalablement enroulés sur des rouleaux de mise en plis. Après avoir laissé agir la composition pendant environ 10 mn, on applique la composition réductrice suivante :

| | |
|---|---|
| - Cystéine | 12,1 g |
| - Sel pentasodique de l'acide diéthylènetriamine penta-acétique | 0,15 g |
| - Ammoniaque q.s. pH 9 | |
| - Parfum q.s. | |
| - Eau déminéralisée q.s.p. | 100 g |

On laisse agir, rince et applique la composition oxydante de l'Exemple 1 selon le même mode de réalisation que décrit à cet exemple.

Après séchage sous casque, les cheveux présentent de belles boucles.

## Revendications

1. Procédé de déformation permanente des cheveux consistant, dans une première étape de réduction, à réaliser l'ouverture des liaisons kératiniques des cheveux par application d'un agent réducteur choisi parmi un amino ou amidothiol ou un de leurs sels puis, dans une deuxième étape d'oxydation, à reformer lesdites liaisons à l'aide d'un composition oxydante ou par action de l'oxygène de l'air, caractérisé par le fait que préalablement ou simultanément à l'application de l'agent réducteur, on applique sur les cheveux, en tant qu'agent dopant dudit agent réducteur, au moins un bromure alcalin, alcalino-terreux ou d'ammonium.

2. Procédé de déformation permanente des cheveux, selon la revendication 1 caractérisé par le fait que le bromure alcalin ou alcalino-terreux est choisi parmi le bromure de sodium, le bromure de potassium et le bromure de calcium.

3. Procédé de déformation permanente des cheveux selon l'une quelconque des revendications précédentes caractérisé par le fait que l'agent dopant est appliqué préalablement à l'étape de réduction, sous la forme d'une composition de pré-traitement, pendant environ 1 à 15 mn, ledit agent dopant étant présent en une concentration comprise entre 1 et 30 % en poids par rapport au poids total de la composition.

4. Procédé de déformation permanente des cheveux selon l'une quelconque des revendications précédentes, caractérisé par le fait que le bromure est appliqué en association avec l'agent réducteur sous forme d'une composition dans laquelle le bromure est présent en une concentration comprise entre 1 et 30 % en poids par rapport au poids total de la composition.

5. Procédé selon l'une quelconque des revendications précédentes pour effectuer une ondulation des cheveux, caractérisé par le fait que la composition réductrice, seule ou en association avec le bromure, est appliquée sur les cheveux préalablement enroulés sur des rouleaux ayant un diamètre de 4 à 20 mm.

6. Procédé selon l'une quelconque des revendications précédentes, pour effectuer le défrisage ou le décrêpage des cheveux, caractérisé par le fait qu'après avoir appliqué la composition réductrice, seule ou en association avec le bromure, on soumet les cheveux à une déformation mécanique par lissage des cheveux.

7. Composition cosmétique pour le premier temps d'une opération de déformation permanente des cheveux, pour la mise en oeuvre du procédé selon les revendications 1 et 2 ou l'une des revendications 4 à 6, caractérisée par le fait qu'elle contient, dans un véhicule cosmétique approprié, en tant que seul et unique agent réducteur au moins un amino ou amidothiol ou un de leurs sels et en tant qu'agent dopant dudit agent réducteur, au moins un bromure alcalin, alcalino-terreux ou d'ammonium.

8. Composition cosmétique selon la revendication 7 caractérisée par le fait que le bromure alcalin ou alcalino-terreux est choisi parmi le bromure de sodium, le bromure de potassium et le bromure de calcium.

9. Composition cosmétique selon l'une quelconque des revendications 7 et 8, caractérisée par le fait que l'agent dopant est présent en une concentration comprise entre 1 et 30 % en poids par rapport au poids total de la composition réductrice.

10. Composition cosmétique selon l'une quelconque des revendications 7 à 9, caractérisée par le fait que le rapport molaire entre l'agent dopant et l'agent réducteur est de préférence compris entre 0,1 et 2,5.

11. Composition cosmétique selon l'une quelconque des revendications 7 à 10, caractérisée par le fait que l'agent réducteur est un amino ou un amidothiol de formule :
HS-A-NR₁R₂ (I)
dans laquelle :
A représente :
(a) le radical divalent -(CH₂)ₙ-, n étant un nombre entier compris entre 2 et 5,
(b) le radical divalent -(CH₂)₂-O-(CH₂)₂-, ou
(c) le radical divalent 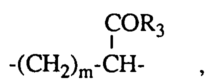 m étant 1 ou 2, R₃ étant (i) le radical OH, (ii) le radical OR₄, R₄ représentant un radical alkyle inférieur, linéaire ou ramifié, ayant de 1 à 3 atomes de carbone ou (iii) le radical -NR₅R₆, R₅ et R₆, identiques ou différents, représentant soit un atome d'hydrogène soit un radical alkyle inférieur, linéaire ou ramifié, ayant de 1 à 3 atomes de carbone,
R₁ et R₂ représentent, indépendamment l'un de l'autre, un atome d'hydrogène, un radical alkyle inférieur, linéaire ou ramifié, ayant de 1 à 4 atomes de carbone, ou un radical -COR₇, R₇ étant un radical alkyle inférieur, linéaire ou ramifié, ayant de 1 à 4 atomes de carbone.

12. Composition cosmétique selon l'une quelconque des revendications 7 à 10, caractérisée par le fait que l'agent réducteur est un amino ou un amidothiol de formule :
HS-(CH₂)ₚ-NR₈R₉ (II)
dans laquelle :
p est 2 ou 3
R₈ représente (a) le radical -CO-(CH₂)ₜ-OH, t étant un nombre entier compris entre 2 et 5,
(b) le radical -CO-(CHOH)₄-CH₂OH, ou
(c) le radical 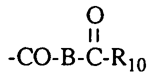 dans lequel R₁₀ représente un radical hydroxy,
B représente (i) le radical divalent (CH₂)_{q}-, q étant 2 ou 3,
(ii) le radical divalent 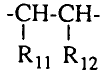 R₁₁ et R₁₂, identiques ou différents,
représentant un radical alkyle, linéaire ou ramifié, ayant de 1 à 4 atomes de carbone ou bien R₁₁ et R₁₂ forment ensemble, avec les atomes de carbone adjacents, un cycle cyclohexane, ou
(iii) le radical divalent 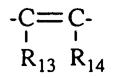 R₁₃ et R₁₄, identiques ou différents,
représentant un atome d'hydrogène, un radical alkyle inférieur, linéaire ou ramifié, ayant de 1 à 4 atomes de carbone, ou bien R₁₃ et R₁₄ forment ensemble, avec les atomes de carbone adjacents, un cycle benzénique, et
R₉ représente un atome d'hydrogène, ou R₉ et R₁₀ pris ensemble forment une liaison simple.

13. Composition cosmétique selon l'une quelconque des revendications 7 à 12, caractérisée par le fait que l'agent réducteur de formule (I) ou (II) est choisi dans le groupe constitué par la cystéine, le cystéinate de méthyle, le cystéinate d'éthyle, la N-acétyl-cystéine, la mercapto-2 éthylamine, la mercapto-3 propylamine, la mercapto-5 pentylamine, la N-acétyl-cystéamine, la N-propionyl-cystéamine, la N-butyryl cystéamine, la N-isobutyryl cystéamine, l'acide N-(mercapto-2 éthyl)succinamique et le N-(mercapto-2 éthyl)succinimide.

14. Composition cosmétique selon l'une quelconque des revendications 7 à 13, caractérisée par le fait que l'agent réducteur est sous forme d'un sel d'aminothiol ou d'amidothiol, ledit sel étant choisi dans le groupe constitué par les chlorhydrates, les bromhydrates, les citrates, les oxalates, les lactates et les acétates.

15. Composition cosmétique selon l'une quelconque des revendications 7 à 14, caractérisée par le fait que l'agent réducteur est présent en une concentration comprise entre 1 et 30 % en poids et de préférence entre 5 et 20 % en poids par rapport au poids total de la composition.

16. Composition cosmétique selon l'une quelconque des revendications 7 à 15, caractérisée par le fait que le pH de la composition est compris entre 4 et 11 et de préférence entre 6 et 10, ledit pH étant obtenu à l'aide d'un agent alcalin choisi parmi l'ammoniaque, la monoéthanolamine, la diéthanolamine, la triéthanolamine, la propanediamine-1,3, un carbonate ou un bicarbonate alcalin ou d'ammonium, un carbonate organique comme le carbonate de guanidine, un hydroxyde alcalin, ou à l'aide d'un agent acidifiant choisi parmi l'acide chlorhydrique, l'acide bromhydrique, l'acide acétique, l'acide lactique, l'acide oxalique ou l'acide borique, ou à l'aide de tampons choisis parmi les phosphates mono et dipotassique.

17. Composition cosmétique selon l'une quelconque des revendications 7 à 16, caractérisée par le fait qu'elle contient en outre un agent tensioactif, un agent traitant, un polymère cationique, un hydrolysat de protéines, un agent de gonflement, un agent renforçant l'efficacité du réducteur, un épaississant, un agent de suspension, un agent opacifiant, un colorant, un parfum ou bien encore un conservateur.

## Claims

1. Process for the permanent-reshaping of hair, consisting, in a first, reduction step, in carrying out opening of the keratin bonds of the hair by application of a reducing agent chosen from an amino- or amidothiol or one of their salts, and then, in a second, oxidation step, in re-forming the said bonds using an oxidizing composition or by the action of aerial oxygen, characterized in that, prior to or simultaneously with the application of the reducing agent, at least one alkali metal, alkaline-earth metal or ammonium bromide is applied to the hair as a doping agent for the said reducing agent.

2. Process for the permanent-reshaping of hair according to Claim 1, characterized in that the alkali metal or alkaline-earth metal bromide is chosen from sodium bromide, potassium bromide and calcium bromide.

3. Process for the permanent-reshaping of hair according to either of the preceding claims, characterized in that the doping agent is applied prior to the reduction step, in the form of a pretreatment composition, for approximately 1 to 15 min, the said doping agent being present at a concentration of between 1 and 30% by weight relative to the total weight of the composition.

4. Process for the permanent-reshaping of hair according to any one of the preceding claims, characterized in that the bromide is applied in combination with the reducing agent in the form of a composition in which the bromide is present at a concentration of between 1 and 30% by weight relative to the total weight of the composition.

5. Process according to any one of the preceding claims, for performing hair waving, characterized in that the reducing composition, alone or in combination with the bromide, is applied to the hair previously coiled on rollers from 4 to 20 mm in diameter.

6. Process according to any one of the preceding claims, for performing hair uncurling or straightening, characterized in that, after the reducing composition, alone or in combination with the bromide, has been applied, the hair is subjected to a mechanical reshaping by smoothing the hair.

7. Cosmetic composition for the first stage of an operation of permanent-reshaping of hair for carrying out the process according to Claims 1 and 2 or one of Claims 4 to 6, characterized in that it contains, in a suitable cosmetic vehicle, at least one amino- or amidothiol or one of their salts as the one and only reducing agent, and at least one alkali metal, alkaline-earth metal or ammonium bromide as a doping agent for the said reducing agent.

8. Cosmetic composition according to Claim 7, characterized in that the alkali metal or alkaline-earth metal bromide is chosen from sodium bromide, potassium bromide and calcium bromide.

9. Cosmetic composition according to either of Claims 7 and 8, characterized in that the doping agent is present at a concentration of between 1 and 30% by weight relative to the total weight of the reducing composition.

10. Cosmetic composition according to any one of Claims 7 to 9, characterized in that the mole ratio of the doping agent to the reducing agent is preferably between 0.1 and 2.5.

11. Cosmetic composition according to any one of Claims 7 to 10, characterized in that the reducing agent is an amino- or an amidothiol of formula:
HS-A-NR₁R₂ (I)
in which:
A represents:
(a) a divalent radical -(CH₂)ₙ-, n being an integer between 2 and 5,
(b) the divalent radical -(CH₂)₂-O-(CH₂)₂-, or
(c) a divalent radical 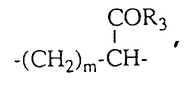 m being 1 or 2 and R₃ being (i) an OH radical, (ii) a radical OR₄, R₄ representing a linear or branched lower alkyl radical having from 1 to 3 carbon atoms or (iii) a radical -NR₅R₆, R₅ and R₆, which may be identical or different, representing either a hydrogen atom or a linear or branched lower alkyl radical having from 1 to 3 carbon atoms,
R₁ and R₂ represent, independently of one another, a hydrogen atom, a linear or branched lower alkyl radical having from 1 to 4 carbon atoms or a radical -COR₇, R₇ being a linear or branched lower alkyl radical having from 1 to 4 carbon atoms.

12. Cosmetic composition according to any one of Claims 7 to 10, characterized in that the reducing agent is an amino- or an amidothiol of formula:
HS-(CH₂)ₚ-NR₈R₉ (II)
in which:
p is 2 or 3
R₈ represents (a) a radical -CO-(CH₂)ₜ-OH, t being an integer between 2 and 5,
(b) the radical -CO-(CHOH)₄-CH₂OH, or
(c) a radical 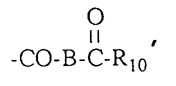 R₁₀ representing a hydroxyl radical,
B represents (i) a divalent radical -(CH₂)_{q}-, q being 2 or 3,
(ii) a divalent radical 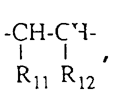 R₁₁ and R₁₂,
which may be identical or different, representing a linear or branched alkyl radical having from 1 to 4 carbon atoms, or alternatively R₁₁ and R₁₂, together with the adjacent carbon atoms, form a cyclohexane ring, or
(iii) a divalent radical 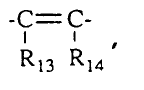 R₁₃ and R₁₄, which may be identical or different, representing a hydrogen atom or a linear or branched lower alkyl radical having from 1 to 4 carbon atoms, or alternatively, R₁₃ and R₁₄, together with the adjacent carbon atoms, form a benzene ring, and
R₉ represents a hydrogen atom, or R₉ and R₁₀ taken together form a single bond.

13. Cosmetic composition according to any one of Claims 7 to 12, characterized in that the reducing agent of formula (I) or (II) is chosen from the group consisting of cysteine, methyl cysteinate, ethyl cysteinate, N-acetylcysteine, 2-mercaptoethylamine, 3-mercaptopropylamine, 5-mercaptopentylamine, N-acetylcysteamine, N-propionylcysteamine, N-butyrylcysteamine, N-isobutyrylcysteamine, N-(2-mercaptoethyl)succinamic acid and N-(2-mercaptoethyl)succinimide.

14. Cosmetic composition according to any one of Claims 7 to 13, characterized in that the reducing agent is in the form of an aminothiol salt or an amidothiol salt, the said salt being chosen from the group consisting of the hydrochlorides, hydrobromides, citrates, oxalates, lactates and acetates.

15. Cosmetic composition according to any one of Claims 7 to 14, characterized in that the reducing agent is present at a concentration of between 1 and 30% by weight, and preferably between 5 and 20% by weight, relative to the total weight of the composition.

16. Cosmetic composition according to any one of Claims 7 to 15, characterized in that the pH of the composition is between 4 and 11, and preferably between 6 and 10, the said pH being obtained using an alkaline agent chosen from aqueous ammonia solution, monoethanolamine, diethanolamine, triethanolamine, 1,3-propanediamine, an alkali metal or ammonium carbonate or bicarbonate, an organic carbonate such as guanidine carbonate or an alkali metal hydroxide, or using an acidifying agent chosen from hydrochloric acid, hydrobromic acid, acetic acid, lactic acid, oxalic acid or boric acid, or using buffers chosen from mono- and dipotassium phosphates.

17. Cosmetic composition according to any one of Claims 7 to 16, characterized in that it contains, in addition, a surfactant, a treatment agent, a cationic polymer, a protein hydrolysate, a swelling agent, an agent for enhancing the efficacy of the reducing agent, a thickener, a suspending agent, an opacifying agent, a colorant, a perfume or alternatively a preservative.

## Patentansprüche

1. Verfahren zur dauerhaften Verformung der Haare (Dauerwelle), das in einem ersten Schritt mittels Reduktion zum Öffnen von Keratinbindungen der Haare durch die Applikation eines Reduktionsmittels besteht, das aus einem Amino- oder Amidothiol oder einem seiner Salze ausgewählt wird, und ferner aus einem zweiten Schritt der Oxidation besteht, wobei die genannten Bindungen mittels einer oxidierenden Zusammensetzung oder durch Luftsauerstoff erneut gebildet werden, dadurch gekennzeichnet, daß zuvor oder gleichzeitig mit der Applikation des Reduktionsmittels auf die Haare mindestens ein Alkalibromid, Erdalkalibromid oder Ammoniumbromid als Dotiermittel des Reduktionsmittels appliziert wird.

2. Verfahren zur dauerhaften Verformung der Haare nach Anspruch 1, dadurch gekennzeichnet, daß das Alkali- oder Erdalkalibromid aus Natriumbromid, Kaliumbromid und Calciumbromid ausgewählt wird.

3. Verfahren zur dauerhaften Verformung der Haare nach einem der vorstehenden Ansprüche, dadurch gekennzeichnet, daß das Dotiermittel vor dem Reduktionsschritt in Form einer Zubereitung zur Vorbehandlung während etwa 1 bis 15 Minute(n) appliziert wird, wobei das Dotiermittel in einer Konzentration zwischen 1 und 30 Gew.-% in bezug auf das Gesamtgewicht der Zusammensetzung vorhanden ist.

4. Verfahren zur dauerhaften Verformung der Haare nach einem der vorstehenden Ansprüche, dadurch gekennzeichnet, daß das Bromid in Kombination mit einem Reduktionsmittel in Form einer Zusammensetzung appliziert wird, worin das Bromid in einer Konzentration zwischen 1 und 30 Gew.-% in bezug auf das Gesamtgewicht der Zubereitung vorhanden ist.

5. Verfahren nach einem der vorstehenden Ansprüche zur Durchführung einer Haarondulierung bzw. Haarkräuselung, dadurch gekennzeichnet, daß die reduzierende Zubereitung alleine oder in Kombination mit dem Bromid auf die zuvor auf Wickler mit einem Durchmesser von 4 bis 20 mm aufgerollten Haare appliziert wird.

6. Verfahren zur Durchführung des Endfrisierens oder Entkräuselns der Haare nach einem der vorstehenden Ansprüche, dadurch gekennzeichnet, daß die Haare nach der Applikation der reduzierenden Zubereitung allein oder in Kombination mit dem Bromid einer mechanischen Verformung durch deren Glätten unterzogen werden.

7. Kosmetische Zubereitung für die erste Phase in einem Vorgang zur dauerhaften Verformung der Haare zur Durchführung des Verfahrens nach den Ansprüche 1 und 2 oder einem der Ansprüche 4 bis 6, dadurch gekennzeichnet, daß sie in einem geeigneten kosmetischen Träger als einzig und alleiniges Reduktionsmittel mindestens ein Amino- oder Amidothiol oder eines deren Salze und als Dotiermittel des genannten Reduktionsmittels mindestens ein Alkalibromid, Erdalkalibromid oder Ammoniumbromid enthält.

8. Kosmetische Zubereitung nach Anspruch 7, dadurch gekennzeichnet, daß das Alkalibromid oder Erdalkalibromid unter Natriumbromid, Kaliumbromid und Calciumbromid ausgewählt ist.

9. Kosmetische Zubereitung nach einem der Ansprüche 7 und 8, dadurch gekennzeichnet, daß das Dotiermittel in einer Konzentration zwischen 1 und 30 Gew.-% in bezug auf das Gesamtgewicht der reduzierenden Zusammensetzung vorhanden ist.

10. Kosmetische Zubereitung nach einem der Ansprüche 7 bis 9, dadurch gekennzeichnet, daß das molare Verhältnis zwischen dem Dotiermittel und dem Reduktionsmittel vorzugsweise zwischen 0,1 und 2,5 beträgt.

11. Kosmetische Zubereitung nach einem der Ansprüche 7 bis 10, dadurch gekennzeichnet, daß das Reduktionsmittel ein Amino- oder Amidothiol der folgenden Formel darstellt:
HS-A-NR₁R₂ (I)
worin
A die folgende Bedeutung aufweist:
(a) als zweiwertiger Rest -(CH₂)ₙ- darstellt, wobei n eine ganze Zahl zwischen 2 und 5 bedeutet, oder
(b) als zweiwertiger Rest -CH₂)₂-O-(CH₂)₂- darstellt, oder
(c) als zweiwertiger Rest 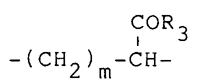 darstellt, wobei m 1 oder 2 bedeutet, und
R₃
(i) die OH-Gruppe, oder
(ii) den Rest OR₄ darstellt, wobei R₄ einen gerad- oder verzweigtkettigen niedrigen Alkylrest mit 1 bis 3 Kohlenstoffatomen bedeutet, oder
(iii) den Rest -NR₅R₆ bedeutet, wobei R₅ und R₆, die gleich oder verschieden sein können, und entweder ein Wasserstoffatom oder einen niedrigen gerad- oder verzweigtkettigen Alkylrest mit 1 bis 3 Kohlenstoffatomen darstellen,
R₁ und R₂ unabhängig voneinander ein Wasserstoffatom, einen niedrigen gerad- oder verzweigtkettigen Alkylrest mit 1 bis 4 Kohlenstoffatomen, oder den Rest
-COR₇ bedeuten, wobei
R₇ einen niedrigen gerad- oder verzweigtkettigen Alkylrest mit 1 bis 4 Kohlenstoffatomen bedeutet.

12. Kosmetische Zubereitung nach einem der Ansprüche 7 bis 10, dadurch gekennzeichnet, daß das Reduktionsmittel ein Amino- oder Amidothiol der folgenden Formel darstellt:
HS-(CH₂)ₚ-NR₈R₉ (II)
worin
p = 2 oder 3 ist,
R₈ die folgende Bedeutung aufweist:
(a) den Rest -CO-(CH₂)ₜ-OH bedeutet, wobei t eine ganze Zahl zwischen 2 und 5 darstellt,
(b) den Rest -CO-(CHOH)₄-CH₂OH bedeutet, oder
(c) den Rest -CO-B-C-R₁₀ bedeutet, worin R₁₀ eine Hydroxygruppe darstellt,
B die folgende Bedeutung aufweist:
(i) den zweiwertigen Rest -(CH₂)_{q}- bedeutet, wobei q = 2 oder 3 ist,
(ii) den zweiwertigen Rest 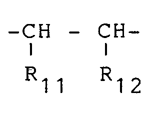 bedeutet, wobei R₁₁ und R₁₂ gleich oder verschieden sein können, und einen gerad- oder verzweigtkettigen Alkylrest mit 1 bis 4 Kohlenstoffatomen bedeuten, oder R₁₁ und R₁₂ zusammengenommen mit den benachbarten Kohlenstoffatomen auch einen Cyclohexanring darstellen, oder
(iii) den zweiwertigen Rest 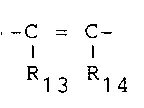 bedeutet, wobei R₁₃ und R₁₄ gleich oder verschieden sein können, und ein Wasserstoffatom, einen niedrigen gerad- oder verzweigtkettigen Alkylrest mit 1 bis 4 Kohlenstoffatomen bedeuten können, oder R₁₃ und R₁₄ auch zusammen mit den benachbarten Kohlenstoffatomen einen Benzolring bilden können, und
R₉ ein Wasserstoffatom bedeutet, oder R₉ und R₁₀ zusammengenommen eine einfache Bindung bilden.

13. Kosmetische Zubereitung nach einem der Ansprüche 7 bis 12, dadurch gekennzeichnet, daß das Reduktionsmittel gemäß der Formel (I) oder (II) aus der aus Cystein, Methylcysteinat, Ethylcysteinat, N-Acetylcystein, 2-Mercaptoethylamin, 3-Mercaptopropylamin, 5-Mercaptopentylamin, N-Acetylcysteamin, N-Propionylcysteamin, N-Butyrylcysteamin, N-Isobutyrylcysteamin, N-(2-Mercaptoethyl)-succinamidsäure und N-(2-Mercaptoethyl)-succinimid bestehenden Gruppe ausgewählt ist.

14. Kosmetische Zubereitung nach einem der Ansprüche 7 bis 13, dadurch gekennzeichnet, daß das Reduktionsmittel in Form eines Aminothiol- oder Amidothiolsalzes vorliegt, wobei dieses Salz aus der aus Chlorhydraten, Bromhydraten, Citraten, Oxalaten, Lactaten und Acetaten bestehenden Gruppe ausgewählt ist.

15. Kosmetische Zubereitung nach einem der Ansprüche 7 bis 14, dadurch gekennzeichnet, daß das Reduktionsmittel in einer Konzentration zwischen 1 und 30 Gew.-%, vorzugsweise zwischen 5 und 20 Gew.-%, in bezug auf das Gesamtgewicht der Zusammensetzung vorhanden ist.

16. Kosmetische Zubereitung nach einem der Ansprüche 7 bis 15, dadurch gekennzeichnet, daß der pH-Wert der Zubereitung zwischen 4 und 11, vorzugsweise zwischen 6 und 10, beträgt, wobei der pH-Wert mittels eines alkalischen Mittels erhalten wird, das aus Ammoniak, Monoethanolamin, Diethanolamin, Triethanolamin, 1,3-Propandiamin, einem Alkali- oder Ammoniumcarbonat oder einem entsprechenden Hydrogencarbonat, oder einem organischen Carbonat wie dem Guanidincarbonat, Alkalihydroxid ausgewählt ist, oder mittels eines Säuerungsmittels erhalten wird, das aus Salzsäure, Bromwasserstoffsäure, Essigsäure, Milchsäure, Oxal- oder Borsäure ausgewählt ist, oder mittels Pufferlösungen eingestellt wird, die aus Mono- oder Dikaliumphosphaten ausgewählt sind.

17. Kosmetische Zubereitung nach einem der Ansprüche 7 bis 16, dadurch gekennzeichnet, daß sie zusätzlich noch ein Tensid, ein Behandlungsmittel, ein kationisches Polymer, ein Proteinhydrolysat, ein Blähmittel, ein Mittel zur Erhöhung der Wirksamkeit des Reduktionsmittels, ein Verdickungsmittel, ein Suspendiermittel, ein opak machendes Mittel, einen Farbstoff, einen Duftstoff oder auch noch ein Konservierungsmittel enthält.
